# FASCICULE DE BREVET EUROPEEN

(11) **EP 3 700 588 B1**
(45) Date de publication et mention de la délivrance du brevet: **23.04.2025**
(21) Numéro de dépôt: 18803323.7
(22) Date de dépôt: 24.10.2018
(51) Int. Cl.: A61L 9/22, H01T 23/00, H01T 19/00, F24F 8/30

(54) **IONISEUR ÉQUIPÉ D'UN ACCÉLÉRATEUR DE FLUX IONIQUE NOTAMMENT POUR LA PROTECTION CONTRE LES MOUSTIQUES**
IONISATOR MIT IONENFLUSSBESCHLEUNIGER, INSBESONDERE ZUM SCHUTZ GEGEN MÜCKEN
IONISER EQUIPPED WITH AN ION-FLUX ACCELERATOR IN PARTICULAR FOR PROTECTION AGAINST MOSQUITOES

(30) Priorité: 28.10.2017 FR 1771136
(43) Date de publication de la demande: 02.09.2020
(73) Titulaire: Saint-Honoré International Diffusion SA, 6821 Rovio (CH)
(72) Inventeur: NOTTON, Philippe, 42230 Roche-La-Moliere (FR); GIOVANNETTI, Christophe, 78240 Chambourcy (FR); LAMAMRI, Salah Eddine, 78240 Chambourcy (FR)
(74) Mandataire: Fédit-Loriot
(86) Numéro de dépôt international: PCT/EP2018/079158
(87) Numéro de publication internationale: WO 2019/081580

(56) Documents cités:
- EP-A1- 0 048 102
- WO-A1-2012/114674
- WO-A1-2017/038111
- US-A1- 2011 150 697

## Description

L'invention a trait au domaine des ioniseurs.

Les ioniseurs sont couramment employés pour purifier l'air. Le principe, assez simple, repose sur l'effet corona : une (ou plusieurs) électrode(s) reliée(s) à une source de haute tension (et faible intensité) électrique provoque(nt) l'ionisation du milieu environnant, accompagnée de décharges électriques.

En pratique, l'électrode peut être formée (au moins à une extrémité libre) de filaments très fins (par ex. en fibre de verre ou de carbone, traités pour être conducteurs de l'électricité). Ces filaments sont placés dans un flux d'air généré par une soufflerie, qui emporte les ions.

Les ions se couplent aux particules en suspension dans l'air, qu'en alourdissant ils contribuent à plaquer au sol.

Un ioniseur typique employant exploitant cette technologie est décrit dans le brevet américain US5268009.

Le document WO2017038111A1 décrit également un dispositif générateur d'ions du type comportant une soufflerie et un générateur d'ions. Le document US2011150697A1 décrit un dispositif de diffusion d'ions.

Il a récemment été découvert qu'un ioniseur peut contribuer à éloigner les insectes (et notamment les diptères) habituellement attirés par les odeurs corporelles (notamment les moustiques femelles). En effet, ces odeurs corporelles sont portées par des particules (notamment la transpiration) qu'il s'avère possible de plaquer au sol par couplage aux ions issus d'un ionisateur.

En d'autres termes, il est possible au moyen d'un ioniseur de désodoriser en permanence l'individu (ou l'animal) exposé aux insectes (notamment aux moustiques). Rendu en quelque sorte invisible à ceux-ci, l'individu (ou l'animal) se trouve protégé de leurs piqûres (ou morsures).

Des ioniseurs ont été proposés pour le port au cou ou au poignet. Leur portée est toutefois limitée et ne peut protéger plusieurs individus simultanément.

Pour augmenter la portée d'un ioniseur, le plus simple est d'augmenter la puissance de la soufflerie. Mais il en résulte une augmentation de la consommation électrique de l'ioniseur, incompatible notamment avec une utilisation prolongée en nomade ou dans des régions dépourvues d'alimentation électrique sur secteur.

Un premier objectif de l'invention est par conséquent de proposer un ioniseur dont la portée soit accrue, tout en présentant une consommation électrique modérée.

Un deuxième objectif de l'invention est de proposer un ioniseur permettant de diffuser des ions dans un volume important, notamment aux fins de rendre, dans ce volume, les individus invisibles au sens olfactif des insectes et notamment des moustiques.

A cet effet, il est proposé un dispositif d'ionisation d'air (ioniseur), qui comprend un boîtier et, dans ce boîtier :
- Une soufflerie, qui inclut un rotor pour générer un flux d'air pulsé et un conduit d'échappement pour canaliser ce flux ;
- Un dispositif de production d'ions, qui comprend :
   - Un générateur de haute tension électrique ;
   - Au moins une électrode raccordée au générateur et dont une extrémité libre, formée d'un bouquet de filaments en matériau conducteur, s'étend au droit du conduit d'échappement pour y libérer des ions par effet Corona ;
- Un diffuseur pourvu :
   - D'une tubulure qui s'étend dans le prolongement du conduit d'échappement et délimite une chambre de compression située en aval des filaments de l'au moins une électrode du dispositif de production d'ions par rapport au flux d'air pulsé généré, et
   - D'un divergent qui prolonge la tubulure et comprend une série de canaux qui s'étendent depuis une face interne du divergent jouxtant la chambre de compression jusqu'à une face externe opposée.

Le diffuseur a pour effet d'accélérer le flux d'air qui le traverse, au bénéfice de la portée (et donc de l'efficacité) de l'ioniseur.

Diverses caractéristiques supplémentaires peuvent être prévues, seules ou en combinaison. Ainsi, par exemple :
- Les canaux vont en s'écartant les uns des autres depuis la face interne du divergent jusqu'à sa face externe.
- Chaque canal va en se rétrécissant depuis la face interne du divergent jusqu'à sa face externe.
- L'ioniseur comprend une bague métallique entourant l'extrémité libre de l'électrode.
- La bague métallique présente une portion évasée (de forme notamment conique ou pyramidale) qui entoure le bouquet de filaments de l'électrode.
- La bague métallique est sertie sur l'électrode.
- La bague métallique est réalisée en cuivre.
- La bague métallique est argentée en surface.
- Le boîtier comprend une embase et un couvercle pourvu de perforations au moins au droit du diffuseur.
- L'ioniseur comprend une carte électronique de commande pourvue d'un interrupteur d'ouverture ou de fermeture d'un circuit électrique d'alimentation du générateur, et le boîtier est pourvu d'un bouton poussoir couplé à l'interrupteur.

D'autres objets et avantages de l'invention apparaîtront à la lumière de la description d'un mode de réalisation, faite ci-après en référence aux dessins annexés dans lesquels :
- la **FIG.1** est une vue en perspective éclatée montrant un dispositif d'ionisation d'air ;
- la **FIG.2** est une vue en perspective éclatée montrant une soufflerie et son diffuseur associé ;
- la **FIG.3** est une vue en arraché partiel, montrant la soufflerie et son diffuseur assemblés ;
- la **FIG.4** est une vue en coupe de la soufflerie et du diffuseur, selon le plan de coupe IV-IV de la **FIG.3** ;
- la **FIG.5** est une vue en coupe selon le plan V-V de la **FIG.4** ;

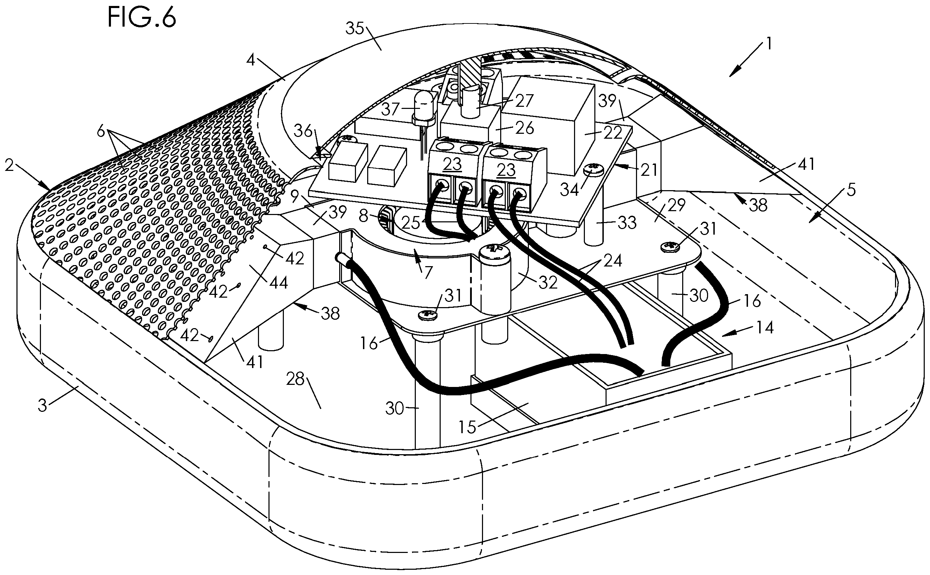

- la **FIG.6** est une vue en arraché partiel montrant le dispositif d'ionisation assemblé.

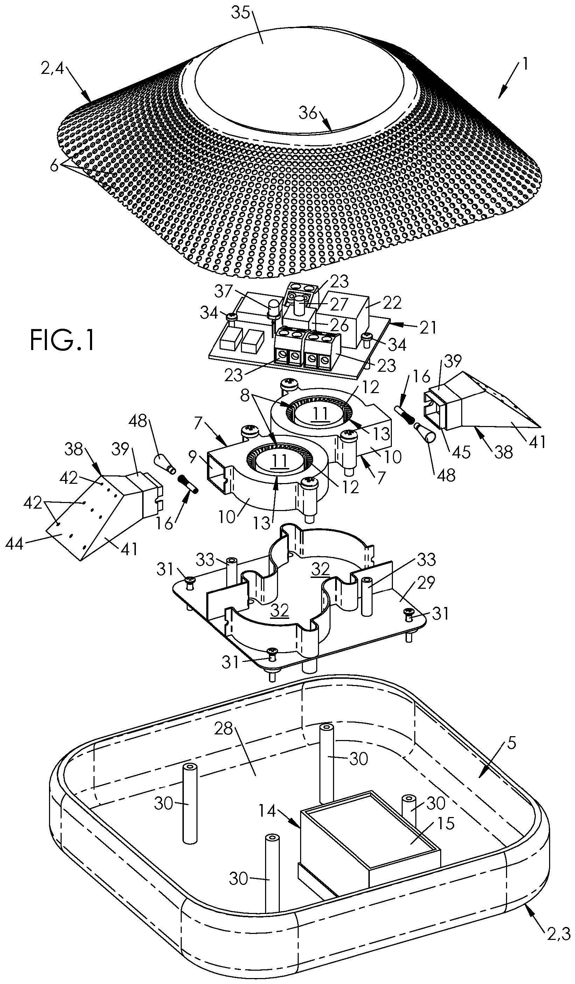

Sur la **FIG.1** est représenté un dispositif d'ionisation d'air, ci-après plus simplement appelé ioniseur **1.** Cet ioniseur **1** est conçu pour produire un ou plusieurs flux d'ions pulsés, destinés à s'associer à des particules en suspension dans l'air de charge électrique opposée.

Les molécules (ou groupes de molécules) formé(e)s par l'association des ions pulsés et des particules sont lourd(e)s et, sous l'effet de leur poids, se trouvent plaqué(e)s au sol.

L'ioniseur **1** est avantageusement conçu pour générer des ions négatifs (anions), typiquement des ions CO₃⁻. Nous verrons ci-après comment.

L'ioniseur 1 comprend, en premier lieu, un boîtier **2.**

Dans l'exemple illustré, le boîtier **2** comprend une embase **3** et un couvercle **4.**

L'embase **3** peut reposer sur un support (par ex. une table), être accrochée à un mur ou une cloison (pose en applique), ou encore être accrochée à un plafond (pose en plafonnier).

Dans l'exemple illustré, l'embase **3** est à contour sensiblement carré (aux coins de préférence arrondis).

Le couvercle **4** est avantageusement pyramidal, à base complémentaire du contour de l'embase **3.**

Ensemble, l'embase **3** et le couvercle **4** définissent un volume **5** interne dans lequel sont disposés divers composants de l'ioniseur **1.**

Le couvercle **4** est pourvu de perforations **6.** Ces perforations **6** sont destinées à permettre le passage de l'air au travers du couvercle **4.**

L'ioniseur **1** comprend, en deuxième lieu, au moins une soufflerie **7.** Cette soufflerie **7** comprend un rotor **8** pour générer un flux d'air pulsé, et un conduit **9** d'échappement pour canaliser ce flux.

Dans l'exemple illustré, la soufflerie **7** se présente sous forme d'un ventilateur. La soufflerie **7** comprend, dans ce cas, un caisson **10** (ici cylindrique) dans lequel le rotor **8** est monté pivotant. Le caisson **10** porte un stator (non représenté) alimenté en courant, et conçu pour générer un champ magnétique tournant entraînant le rotor **8** en rotation.

Dans l'exemple illustré, le rotor **8** comprend un arbre **11** et une pluralité d'ailettes **12** périphériques solidaires de l'arbre **11** et qui, lors de la rotation du rotor **8,** génèrent le flux d'air.

Selon un mode préféré de réalisation illustré sur la **FIG.1****,** l'ioniseur **1** comprend deux souffleries **7.** Les souffleries **7** sont par ex. montées dos-à-dos, c'est-à-dire que les flux générés sont pulsés dans des directions opposées.

Comme illustré notamment sur la **FIG.1****,** le caisson **10** est pourvu d'une entrée d'air (ici formée par une ouverture **13** à l'aplomb du rotor **8).**

Le conduit **9** d'échappement est ici formé par une ouverture pratiquée sur un côté du caisson **10.** Dans l'exemple illustré, le conduit **9** d'échappement est à section rectangulaire (ou carrée) mais il pourrait être à section circulaire ou ovale.

L'ioniseur **1** comprend, en troisième lieu, un dispositif **14** de production d'ions, qui comprend :
- Un générateur **15** de haute tension électrique ;
- Au moins une électrode **16** raccordée au générateur **15** et dont une extrémité **16** libre, formée d'un bouquet de filaments **18** en matériau conducteur, s'étend dans le conduit **9** d'échappement pour y libérer des ions par effet corona.

Le générateur **15** de haute tension est raccordé à une source de tension électrique (par ex. une batterie, une pile, ou le secteur - via un transformateur). Le générateur **15** est conçu pour produire de l'électricité à haute tension (plusieurs milliers de volts, voire plusieurs dizaines de milliers de volts) et faible intensité (quelques milliampères, voire quelques microampères).

Dans l'exemple illustré, où l'ioniseur **1** comprend deux souffleries 7, le dispositif **14** de production d'ions comprend deux électrodes **16,** chacune associée à une soufflerie **7.**

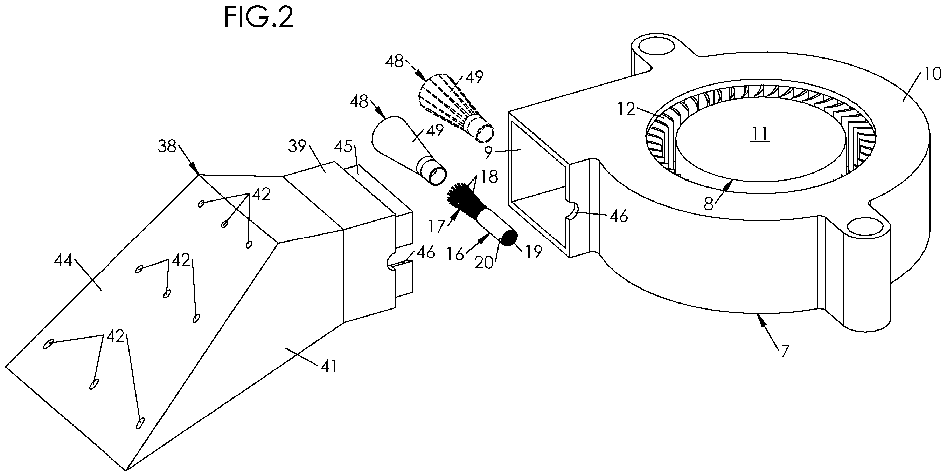

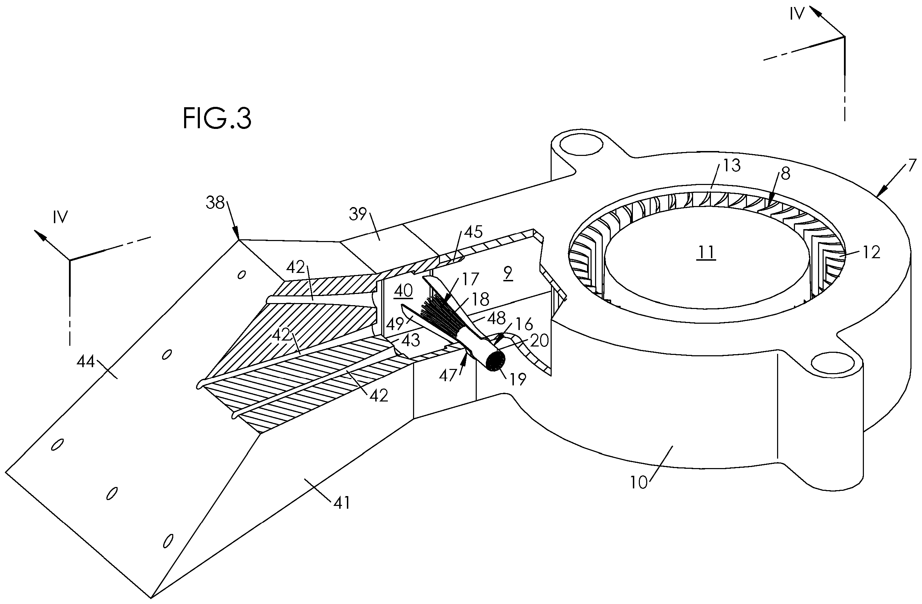

Chaque électrode **16** comprend un conducteur **19** raccordé au générateur. Pour minimiser les pertes, le conducteur **19** est, sur une partie au moins de sa longueur, entouré d'une gaine **20** (par ex. dans un polymère isolant souple). Comme cela est bien visible sur la **FIG.2** et sur la **FIG.3****,** l'extrémité **16** libre de l'électrode (c'est-à-dire le bouquet de filaments **18)** est dénudée.

Les filaments **18** peuvent constituer ensemble le conducteur **19** sur toute sa longueur, ou ne former qu'une partie du conducteur **19** en étant raccordées à un fil (par ex. métallique) lui-même relié au générateur **15.**

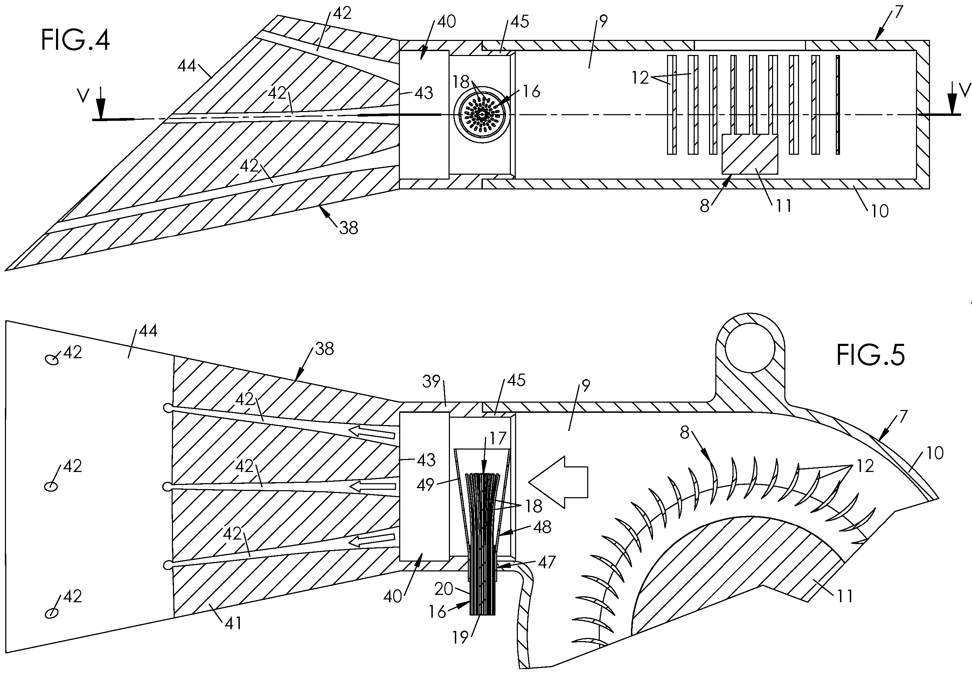

Comme on le voit sur la **FIG.1** et sur la **FIG.5****,** l'ioniseur **1** comprend, en quatrième lieu, une électronique de commande, sous forme d'une carte **21** de circuits intégrés incluant des composants électroniques et formant un circuit électrique d'alimentation du générateur **15.**

Dans l'exemple illustré, la carte **21** comprend un transformateur **22,** conçu pour générer une tension moyenne (de l'ordre de 12 V) destinée à alimenter les autres composants et à être fournie au générateur **15** de haute tension.

La carte **21** comprend également des borniers **23,** sur lesquels sont branchés des conducteurs **24** électriques d'alimentation du générateur **15,** ainsi que des conducteurs **25** électriques d'alimentation de la (ou de chaque) soufflerie **7.** Plus précisément, ces derniers conducteurs **25** électriques sont raccordés au stator de chaque soufflerie **7.**

La mise en route de l'ioniseur **1** peut être commandée à distance, par ex. au moyen d'une télécommande (à infrarouge ou à radiofréquences), ou au moyen d'un interrupteur distant monté sur un circuit électrique d'alimentation de l'ioniseur **1.**

La mise en route de l'ioniseur **1** peut également être effectuée par action directe sur celui-ci. Ainsi, selon un mode de réalisation illustré sur les **FIG.1** **et** **FIG.5****,** la carte **21** est pourvue d'un interrupteur **26** d'ouverture ou de fermeture du circuit électrique d'alimentation du générateur **15.**

Cet interrupteur **26** comprend par ex. une tige **27** montée en translation entre une position haute dans laquelle le circuit est ouvert (le générateur **15** et chaque soufflerie **7** étant alors privés d'alimentation électrique) et une position basse dans laquelle le circuit est fermé (le générateur **15** et chaque soufflerie **7** étant alors alimentés électriquement).

D'un point de vue architectural, comme illustré sur la **FIG.1** et sur la **FIG.5****,** le générateur **15** est monté sur une paroi **18** de fond de l'embase **3.** La (ou chaque) soufflerie **7** est montée sur une platine **29** (réalisée par ex. dans une matière plastique). Cette platine **29** est fixée sur l'embase **3.** Dans l'exemple illustré, la platine **29** est montée sur des cheminées **30** qui font saillie de la paroi **28** de fond de l'embase **3.** La fixation de la platine **29** sur les cheminées **30** est par ex. réalisée au moyen de vis **31.**

Comme illustré sur la **FIG.1****,** chaque soufflerie **7** est emboîtée dans un logement **32** complémentaire formé en saillie sur la platine **29.** La fixation de chaque soufflerie **7** sur la platine **29** est par ex. réalisée au moyen de vis.

Selon un mode préféré de réalisation illustré sur la **FIG.1** et sur la **FIG.5****,** la carte **21** électronique est montée par-dessus la (ou les) soufflerie(s). A cet effet, la carte **21** est montée sur des pions **33** qui font saillie de la platine **29.** La fixation de la carte **21** sur les pions **33** est par ex. réalisée au moyen de vis **34.**

Dans l'exemple illustré, le couvercle **4** se présente sous forme d'une pyramide tronquée et est surmonté d'un cache **35,** de forme par ex. hémisphérique.

Un interstice **36** est avantageusement prévu entre le pourtour du cache **35** et le couvercle **4.**

Cet interstice **36** peut, d'abord, former une entrée d'air pour alimenter en air frais la (ou les) soufflerie(s) **7.**

L'interstice **36** peut, ensuite, former un passage pour la lumière émise par un témoin lumineux de marche de l'ioniseur **1,** par ex. une diode **37** électroluminescente (DEL ou LED) montée sur la carte **21** électronique de commande.

Selon un mode de réalisation préféré illustré sur les **FIG.1** et **FIG.5****,** dans lequel la mise en route de l'ioniseur **1** est effectuée par une action manuelle directe sur celui-ci, le cache **35** forme un bouton poussoir couplé à l'interrupteur **26.** L'actionnement du cache **35** (par simple appui du doigt ou de la main) permet, par l'intermédiaire de la tige **27** de l'interrupteur **26,** d'ouvrir ou de fermer le circuit électrique d'alimentation du générateur **15** et de la (des) soufflerie(s). Selon un mode de réalisation illustré sur la **FIG.5****,** le cache **35** est emmanché à force sur la tige **27,** qui en assure ainsi le support.

En fonctionnement, le (ou chaque) rotor **8** génère un flux d'air pulsé au travers du conduit **9** d'échappement. Les filaments **18** de l'électrode **16** (alimentée en haute tension par le générateur **15)** génèrent des décharges électriques (effet corona) accompagnées d'une production d'ions en grande quantité. Ces ions sont emportés par le flux d'air et éjectés hors de l'ioniseur **1** au travers des perforations **6** du couvercle **4.**

Pour augmenter la portée de l'ioniseur **1,** c'est-à-dire la distance à laquelle les ions sont projetés, l'ioniseur **1** comprend, en cinquième lieu, et pour chaque soufflerie **7,** un diffuseur **38.**

Ce diffuseur **38** est pourvu :
- D'une tubulure **39** qui s'étend dans le prolongement du conduit **9** d'échappement et délimite une chambre **40** de compression, et
- D'un divergent **41** qui prolonge la tubulure **39** et comprend une série de canaux **42** qui s'étendent depuis une face **43** interne du divergent **41** jouxtant la chambre **40** de compression jusqu'à une face **44** externe opposée.

Dans l'exemple illustré, le diffuseur **38** est une pièce monobloc, de préférence réalisée dans une matière plastique.

La tubulure **39** présente une section identique ou complémentaire du conduit **9** d'échappement. Dans l'exemple illustré, la tubulure **39** est ainsi à section rectangulaire (ou carrée).

Le diffuseur **38** peut être fixé directement sur le caisson **10** de la soufflerie **7.** Dans l'exemple illustré, le diffuseur **38** est pourvu d'un embout **45** mâle qui fait saillie de la tubulure pour venir s'emboîter à force (ou avec encliquetage) dans le conduit **9** d'échappement.

Comme illustré sur la **FIG.2****,** le diffuseur **38** et/ou la soufflerie **7** est (sont) pourvue(s) d'une échancrure **46** pour permettre le montage **(****FIG.2****)** et le passage **(****FIG.3****)** de l'électrode **16,** de manière que le bouquet de filaments **18** s'étende au droit du conduit **9** d'échappement. Dans l'exemple illustré, le diffuseur **38** et le caisson **10** de la soufflerie **7** sont tous deux pourvus d'échancrures **46** complémentaires qui, une fois le diffuseur **38** monté sur le caisson **10,** forment conjointement une ouverture **47** au travers de laquelle passe l'électrode **16,** dont les filaments **18** s'étendent ainsi à la jonction entre le conduit **9** d'échappement et la tubulure **39.**

Dans l'exemple illustré (cf. notamment **FIG.3****),** les canaux **42** sont au nombre de neuf, mais ce nombre n'est qu'illustratif. Les canaux **42** pourraient être moins nombreux, ou plus nombreux.

Les canaux **42** vont de préférence en s'écartant les uns des autres depuis la face **43** interne du divergent **41** jusqu'à sa face **44** externe. La somme des sections de passage des canaux **42** est inférieure à la section de passage du conduit **9** d'échappement (et de la tubulure **39**). Par conséquent, l'air est comprimé dans la chambre **40** de compression, en amont et au voisinage de la face **43** interne du divergent **41.**

Il en découle que la vitesse de déplacement de l'air (chargé d'ions) est supérieure dans les canaux **42** à la vitesse de déplacement de l'air dans le conduit **9** d'échappement (l'écoulement de l'air est illustré par des flèches blanches sur la **FIG.5****).**

En d'autres termes, le diffuseur **38** procure, dans le divergent **41,** une accélération du flux d'air entrant par la face **43** interne. Il en résulte que les ions sont projetés hors de l'ioniseur **1** à une distance supérieure à la distance à laquelle ils seraient projetés sans le diffuseur **38.** La portée (et donc l'efficacité) de l'ioniseur **1** s'en trouve accrue.

En outre, selon un mode préféré de réalisation, chaque canal **42** va en se rétrécissant depuis la face **43** interne du divergent **41** jusqu'à sa face **44** externe, ce qui augmente encore l'effet d'accélération du flux et donc la portée de l'ioniseur **1.**

Les perforations **6** dans le couvercle **4** s'étendent au moins au droit du (ou de chaque) diffuseur **38.** On veillera à ce que chaque canal **42** débouche au droit d'une perforation **6,** de sorte à minimiser les rebonds du flux d'air sortant. Dans l'exemple illustré, des perforations **6** sont prévues sur toute la surface du couvercle **4.**

La face **44** externe du divergent s'étend de préférence au plus près du couvercle **4.** Selon un mode préféré de réalisation, la face **44** externe du divergent **41** épouse l'intérieur du couvercle **4.**

Pour augmenter la densité d'ions produits au niveau des filaments **18,** l'ioniseur **1** peut comprendre une bague **48** métallique entourant l'extrémité **17** libre de l'électrode **16.**

Cette bague **48,** maintenue à un potentiel nul, forme une masse électrique qui procure un effet de gain en accroissant le nombre de décharges électriques (et donc la formation d'ions) au niveau de l'extrémité **17** libre de l'électrode **16.**

Selon un mode préféré de réalisation illustré sur les **FIG.2** et **FIG.3****,** la bague **48** métallique présente une portion **49** évasée qui entoure le bouquet de filaments **18** de l'électrode **16.** Cette portion **49** évasée a pour fonction d'épouser le bouquet de filaments **18,** qui ont en effet tendance à s'écarter les uns des autres. Selon un mode de réalisation illustré en trait plein sur la **FIG.2****,** la portion **49** évasée de la bague est conique. Selon un autre mode de réalisation, illustré en pointillés sur la **FIG.2****,** la portion **49** évasée est pyramidale (à base polygonale) et présente un nombre de facettes quelconque (quoique supérieur ou égal à trois).

La bague **48** métallique est avantageusement sertie sur l'électrode **16.** Comme illustré sur la **FIG.3****,** la bague **48** métallique est de préférence emmanchée dans l'ouverture **47.** La bague **48** métallique peut être réalisée en cuivre. En variante, la bague métallique est argentée en surface. Elle peut se présenter sous forme d'une tresse ou d'un treillis.

## Revendications

1. Dispositif (**1**) d'ionisation d'air, qui comprend un boîtier **(2)** et, dans ce boîtier **(2) :**
- Une soufflerie **(7),** qui comprend un rotor **(8)** pour générer un flux d'air pulsé et un conduit **(9)** d'échappement pour canaliser ce flux ;
- Un dispositif **(14)** de production d'ions, qui comprend :
• Un générateur **(15)** de haute tension électrique ;
• Au moins une électrode **(16)** raccordée au générateur **(15)** et dont une extrémité **(17)** libre, formée d'un bouquet de filaments **(18)** en matériau conducteur, s'étend au droit du conduit **(9)** d'échappement pour y libérer des ions par effet corona ;
Ce dispositif **(1)** étant **caractérisé en ce qu'**il comprend en outre :
- Un diffuseur **(38)** pourvu :
• D'une tubulure **(39)** qui s'étend dans le prolongement du conduit **(9)** d'échappement et délimite une chambre **(40)** de compression située en aval des filaments de l'au moins une électrode du dispositif de production d'ions par rapport au flux d'air pulsé généré, et
• D'un divergent (**41**) qui prolonge la tubulure **(39)** et comprend une série de canaux **(42)** qui s'étendent depuis une face **(43)** interne du divergent **(41)** jouxtant la chambre **(40)** de compression jusqu'à une face **(44)** externe opposée.

2. Dispositif **(1)** selon la revendication 1, **caractérisé en ce que** les canaux **(42)** vont en s'écartant les uns des autres depuis la face **(43)** interne du divergent (**41**) jusqu'à sa face **(44)** externe.

3. Dispositif **(1)** selon la revendication 1 ou la revendication 2, **caractérisé en ce que** chaque canal **(42)** va en se rétrécissant depuis la face **(43)** interne du divergent (**41**) jusqu'à sa face **(44)** externe.

4. Dispositif **(1)** selon l'une des revendications précédentes, **caractérisé en ce qu'**il comprend une bague **(48)** métallique entourant l'extrémité **(17)** libre de l'électrode **(16).**

5. Dispositif (**1**) selon la revendication 4, **caractérisé en ce que** la bague **(48)** métallique présente une portion **(49)** évasée qui entoure le bouquet de filaments **(18)** de l'électrode **(16).**

6. Dispositif **(1)** selon la revendication 4 ou la revendication 5, **caractérisé en ce que** la bague **(48)** métallique est sertie sur l'électrode **(16).**

7. Dispositif **(1)** selon l'une des revendications 4 à 6, **caractérisé en ce que** la bague **(48)** métallique est réalisée en cuivre.

8. Dispositif (**1**) selon l'une des revendications 4 à 6, **caractérisé en ce que** la bague **(48)** métallique est argentée en surface.

9. Dispositif **(1)** selon l'une des revendications précédentes, **caractérisé en ce que** le boîtier **(2)** comprend une embase **(3)** et un couvercle **(4)** pourvu de perforations **(6)** au moins au droit du diffuseur **(38).**

10. Dispositif **(1)** selon l'une des revendications précédentes, **caractérisé en ce qu'**il comprend une carte **(21)** électronique de commande pourvue d'un interrupteur **(26)** d'ouverture ou de fermeture d'un circuit électrique d'alimentation du générateur **(15),** et **en ce que** le boîtier **(2)** est pourvu d'un bouton **(35)** poussoir couplé à l'interrupteur **(26).**

## Patentansprüche

1. Luftionisierungsvorrichtung **(1),** die ein Gehäuse **(2)** umfasst und, in diesem Gehäuse **(2):**
- Ein Gebläse **(7),** das einen Rotor **(8)** zur Erzeugung eines gepulsten Luftstroms und einen Abluftkanal **(9)** zur Kanalisierung dieses Luftstroms umfasst;
- Ionenerzeugungsvorrichtung **(14),** die Folgendes umfasst:
• ein elektrischer Hochspannungsgenerator **(15);**
• Mindestens eine Elektrode **(16),** die an den Generator **(15)** angeschlossen ist und deren freies Ende **(17)** aus einem Bündel von Filamenten **(18)** aus leitfähigem Material besteht, sich rechts vom Abgaskanal **(9)** erstreckt, um dort durch Corona-Effekt Ionen freizusetzen;
Diese Vorrichtung **(1)** ist **dadurch gekennzeichnet, dass** sie ferner Folgendes umfasst:
- einen Diffusor **(38)** mit:
• einem Rohr **(39),** das sich in der Verlängerung des Auslasskanals **(9)** erstreckt und eine den Filamenten der mindestens einen Elektrode der Ionenerzeugungsvorrichtung nachgeschaltete Kompressionskammer **(40)** in Bezug auf den erzeugten gepulsten Luftstrom begrenzt, und
• einem Divergenten **(41),** der den Schlauch **(39)** verlängert und eine Reihe von Kanälen **(42)** umfasst, die sich von einer inneren Seite **(43)** des Divergenten **(41)** neben der Kompressionskammer **(40)** bis zu einer gegenüberliegenden äußeren Seite **(44)** erstrecken.

2. Vorrichtung **(1)** nach Anspruch 1, **dadurch gekennzeichnet, dass** die Kanäle **(42)** von der inneren Seite **(43)** des Divergenten **(41)** bis zu seiner äußeren Seite **(44)** auseinander verlaufen.

3. Vorrichtung (1) nach Anspruch 1 oder Anspruch 2, **dadurch gekennzeichnet, dass** sich jeder Kanal **(42)** von der inneren Seite **(43)** des Divergenten **(41)** bis zu seiner äußeren Seite **(44)** verengt.

4. Vorrichtung **(1)** nach einem der vorhergehenden Ansprüche, **dadurch gekennzeichnet, dass** sie einen Metallring **(48)** umfasst, der das freie Ende **(17)** der Elektrode **(16)** umgibt.

5. Vorrichtung **(1)** nach Anspruch 4, **dadurch gekennzeichnet, dass** der Metallring **(48)** einen aufgeweiteten Teil **(49)** aufweist, der den Filamentbündel **(18)** der Elektrode **(16)** umgibt.

6. Vorrichtung **(1)** nach Anspruch 4 oder Anspruch 5, **dadurch gekennzeichnet, dass** der Metallring **(48)** auf die Elektrode **(16)** aufgepresst ist.

7. Vorrichtung **(1)** nach einem der Ansprüche 4 bis 6, **dadurch gekennzeichnet, dass** der Metallring **(48)** aus Kupfer gefertigt ist.

8. Vorrichtung **(1)** nach einem der Ansprüche 4 bis 6, **dadurch gekennzeichnet, dass** der Metallring **(48)** an der Oberfläche silberfarben ist.

9. Vorrichtung **(1)** nach einem der vorhergehenden Ansprüche, **dadurch gekennzeichnet, dass** das Gehäuse **(2)** einen Sockel **(3)** und einen Deckel **(4)** mit Perforationen **(6)** mindestens rechts vom Diffusor **(38)** umfasst.

10. Vorrichtung **(1)** nach einem der vorhergehenden Ansprüche, **dadurch gekennzeichnet, dass** sie eine elektronische Steuerkarte **(21)** mit einem Schalter **(26)** zum Öffnen oder Schließen eines Stromversorgungs-Stromkreises des Generators **(15)** umfasst und, dass das Gehäuse **(2)** mit einem mit dem Schalter **(26)** gekoppelten Druckknopf **(35)** versehen ist.

## Claims

1. Device (1) for ionising air, which comprises a case (2) and, in this case (2):
- A blower (7), which comprises a rotor (8) to generate a pulsed airflow and an exhaust pipe (9) to channel this flow;
- A device (14) for producing ions, which comprises:
• A high-voltage electrical generator (15);
• At least one electrode (16) connected to the generator (15) and of which a free end (17) formed of a cluster of filaments (18) made of conductive material, extends to the right of the exhaust pipe (9) to release ions there by corona discharge;
This device (1) being **characterised in that** it further comprises:
- A diffuser (38) provided:
• With a pipe (39) which extends into the extension of the exhaust pipe (9) and delimits a compression chamber (40) located downstream of the filaments of the at least one electrode of the ion-generating device with respect to the pulsed air flow generated, and
• With an expander (41) which extends the pipe (39) and comprises a series of channels (42) which extend from an inner face (43) of the expander (41) adjoining the compression chamber (40) to an opposite outer face (44).

2. Device (1) according to claim 1, **characterised in that** the channels (42) will be deviated from one another from the inner face (43) of the expander (41) to the outer face (44) thereof.

3. Device (1) according to claim 1 or claim 2, **characterised in that** each channel (42) will be narrowed from the inner face (43) of the expander (41) to the outer face (44) thereof.

4. Device (1) according to one of the preceding claims, **characterised in that** it comprises a metal ring (48) surrounding the free end (17) of the electrode (16).

5. Device (1) according to claim 4, **characterised in that** the metal ring (48) has a flared portion (49) which surrounds the cluster of filaments (18) of the electrode (16).

6. Device (1) according to claim 4 or claim 5, **characterised in that** the metal ring (48) is crimped on the electrode (16).

7. Device (1) according to one of claims 4 to 6, **characterised in that** the metal ring (48) is made of copper.

8. Device (1) according to one of claims 4 to 6, **characterised in that** the metal ring (48) is silver on the surface.

9. Device (1) according to one of the preceding claims, **characterised in that** the case (2) comprises a base (3) and a cover (4) provided with perforations (6) at least to the right of the diffuser (38).

10. Device (1) according to one of the preceding claims, **characterised in that** it comprises an electronic control board (21) provided with a switch (26) for opening or closing a circuit for electrically supplying the generator (15), and **in that** the case (2) is provided with a pushbutton (35) coupled with the switch (26).
